# EUROPEAN PATENT SPECIFICATION

(11) **EP 1 871 488 B1**
(45) Date of publication and mention of the grant of the patent: **06.06.2018**
(21) Application number: 06749628.1
(22) Date of filing: 11.04.2006
(51) Int. Cl.: A62B 9/00

(54) **SUPPLIED AIR RESPIRATOR THAT HAS AN ADJUSTABLE LENGTH HOSE**
UMLUFTUNABHÄNGIGES ATEMSCHUTZGERÄT MIT SCHLAUCH MIT VERSTELLBARER LÄNGE
RESPIRATEUR À AIR FOURNI POURVU D'UN FLEXIBLE AJUSTABLE EN LONGUEUR

(30) Priority: 15.04.2005 US 106833
(43) Date of publication of application: 02.01.2008
(73) Proprietor: 3M Innovative Properties Company, St. Paul, MN 55133-3427 (US)
(72) Inventor: WALKER, Garry J., Saint Paul, Minnesota 55133-3427 (US); CURRAN, Desmond T., Saint Paul, Minnesota 55133-3427 (US); MURPHY, Andrew, Saint Paul, Minnesota 55133-3427 (US); PARKIN, Derek A., Saint Paul, Minnesota 55133-3427 (US)
(74) Representative: Herzog, Fiesser & Partner Patentanwälte PartG mbB
(86) International application number: PCT/US2006/013271
(87) International publication number: WO 2006/113203

(56) References cited:
- DE-C- 702 098
- DE-C- 882 185
- US-A- 2 733 734
- US-A- 2 999 497
- US-A1- 2004 065 335
- US-A1- 2004 200 921
- US-A1- 2005 126 565
- US-B1- 6 889 688

## Description

The present invention pertains to a supplied air respirator that uses an adjustable length hose as a conduit between the respirator facepiece and the clean air supply source.

### BACKGROUND

Supplied air respirators are regularly worn in environments where the surrounding air contains contaminants. Clean air is delivered to the wearer from a supply tank or from a powered air source that drives the ambient air through an air filter.

Systems that use a powered air source to supply clean air to the wearer are referred to as powered air purifying respirators - known shorthand as "PAPRs". PAPRs typically have two main parts: a facepiece and a filtering unit. The facepiece is worn at least over the nose and mouth of the user (it also may cover the eyes and ears), and the filtering unit is commonly worn about the user's waist. The filtering unit often includes filter cartridges, a housing, a fan, and an electric motor that drives the fan. The fan and motor are contained within the housing, and the filter cartridges are attached to the housing body. Ambient air is filtered by being forced through filter elements that are contained within the filter cartridges. This filtered air is then delivered to the facepiece through a fixed length hose. The electrically powered fan drives the air from the filter cartridges, through the hose, and into the facepiece interior. Because the fan does the work required for air movement through the PAPR system, the user is able to comfortably receive a clean supply of air with little effort. Representative examples of known PAPRs are described in the following patents: U.S. Patent 6,796,304 to Odell et al. U.S. Patent 6,575,165 to Cook et al., U.S. Patent 6,666,209 to Bennett et al., and U.S. Patent 6,837,239 to Beizndtsson et al.

Supplied air respirators that use a pressurized supply tank to provide clean air to the wearer are frequently referred to as self-contained breathing apparatus, or as "SCBAs". SCBAs also have a hose that delivers clean air to the face piece from the clean air supply source. Examples of SCBAs are shown in the following publications: US 2005/0022817 A1, US 2004/0182395 A1, and US 2003/0111076 A1.

US 2,999,497 A discloses a breathing apparatus hose restrainer.

DE 882 185 C discloses a device for adjusting the length of a corrugated tube for a respiratory protective device.

US 2004/200921 A1 discloses a hose dispenser.

While known supplied air systems have been very beneficial in supplying clean air to a worker, these known products have had one particular drawback: the hose that is used to deliver the air to the facepiece frequently gets caught on projections that exist in the workplace where the supplied air respirator is used. Supplied air respirators are designed to be used by persons of various sizes. Thus, the hose that extends from the filtering unit to the facepiece is sized to be long enough to accommodate persons that have long torsos. When a person who has a shorter torso, however, uses the same supplied air respirator, there is an extra hose length that dangles away from the user's body. This extra hose can become caught on articles that project into the area where the worker operates, making the user irritated and perhaps creating unsafe conditions in the work environment. Even more critically, a hose that is caught on an object may cause disengagement of the hose from either the face piece or the clean air supply source, thus compromising the breathing protection furnished by the system. The tangled hose also can cause adjacent objects to be knocked over or may cause the user to lose balance.

The problem of extra hose length has been dealt with in the art providing the end users with hoses of various lengths. By offering more that one length of hose, the user can select the hose that is most suitable to them. This approach, however, also is disadvantageous because it requires the making and distribution of more than one hose. The need for multiple hoses increases the cost and complexity of the employer's health and safety program. And even when the users are furnished with more than one length hose, the user still has to chose the one nearest to their requirements, rather than having one of a precise length.

### SUMMARY OF THE INVENTION

The present invention provides a supplied air respirator according to claim 1. The present invention differs from known supplied air respirators in that it uses a sleeve as hose length adjustment means. This feature is beneficial in that it allows persons of various heights and sizes to wear the same supplied air respirator without encountering hose entanglement problems. There is no need to have multiple sized hoses available for distribution, and each hose can be tailored to a precise length. A user of the inventive supplied air respirator can fashion the hose length to accommodate the length of their torso and thus preclude extra hose from dangling freely from their body. The inventive supplied air respirator thus provides greater convenience and safety to the end user and to other persons and things in the work environment.

These and other advantages of the invention are more fully shown and described in the drawings and detailed description of this invention, where like reference numerals are used to represent similar parts. It is to be understood, however, that the drawings and description are for the purposes of illustration only and should not be read in a manner that would unduly limit the scope of this invention.

### Glossary

In describing this invention, the following terms are defined as set forth below:
"adjustable length hose" means a hose that can be adjusted to a desired length;
"breathing zone" means the portion of an interior gas space were clean air is inhaled by a wearer of a supplied air respirator;
"clean air" means air (or other oxygen-containing gas) that has been filtered or that has otherwise been made safe to breathe for providing oxygen to a person;
"clean air supply source" means an apparatus, such as a filtering unit or tank, that is capable of providing a supply of clean air (or oxygen) for person to safely breathe;
"compliant face contacting member" means the portion of a face piece that is compliantly fashioned for allowing the mask body to be comfortably supported over a person's nose and mouth;
"extends or extends from" means that the hose is located somewhere between the clean air supply source and the face piece to assist in allowing fluid communication to occur between such parts (the hose does not need to be directly attached at either end to such parts);
"exterior gas space" means the ambient atmospheric gas space that surrounds a face piece when worn on a person and that ultimately receives exhaled gas after it exits the interior gas space of a mask;
"face piece" means a device that is worn by a person over at least the respiratory passages (nose and mouth) of a person to help create an interior gas space separate from and exterior gas space;
"filter cartridge" means a structure that includes a filter element and that is adapted for connection to or use in a filtering unit;
"filtering unit" means the portion of a PAPR that is responsible for filtering ambient air and causing powered air movement;
"hose" means a device that includes a conduit that has a fluid in permeable wall(s) through which air can travel for delivering clean air from a clean air supply source to a face piece;
"interior gas space" means the space that exists between a mask body and a person's face when the mask is being worn;
"mask body" means a structure that can fit at least over the nose and mouth of a person and that can help define an interior gas space separate from an exterior gas space;
"powered air purifying respirator or PAPR" means a supplied air respirator that uses an external power source to deliver filtered air to the breathing zone of an interior gas space;
"self-containing breathing apparatus" or SCBA means a supplied air respirator that has a pressurized bottle or tank in which a supply of clean air is stored; and
"supplied air respirator" means a device that is capable of delivering a supply of clean air to a wearer of the device.

### BRIEF DESCRIPTION OF THE DRAWINGS

FIG. 1 is a perspective view of a powered air purifying respirator system **10** in accordance with the present invention;
FIG. 2 is a plan view of an adjustable length hose **12** in accordance with the present invention, which hose **12** includes a sleeve **14** for fixing the tube length;
FIG. 3 is a plan view of the adjustable length hose **12** of FIG. 2, including sleeves **14, 38** for covering the whole tube length;
FIG. 4 is a plan view of an adjustable length hose **12'** that has a first portion **35** that is fixed in length or unextended; and
FIG. 5 is a perspective on a self-contained breathing apparatus **50** in accordance with the present invention.

### DETAILED DESCRIPTION OF THE PREFERRED EMBODIMENTS

In describing preferred embodiments of the invention, specific terminology is used for the sake of clarity. The invention, however, is not intended to be limited to the specific terms so selected, and it is to be understood that each term so selected includes all technical equivalents that operate similarly.

In the practice of the present invention, a powered air purifying respirator is provided that not as unwieldy as supplied air respirators heretofore known in the art. Unlike known supplied air respirators, which had hoses of uniform length and which could be too long for smaller users, the present invention furnishes the art with a supplied a respirator that has an adjustable length hose that reduces problems and costs associated with unnecessary hose length.

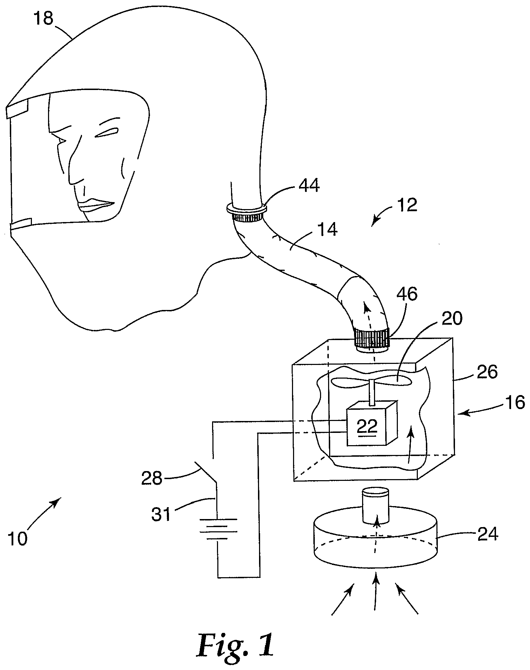

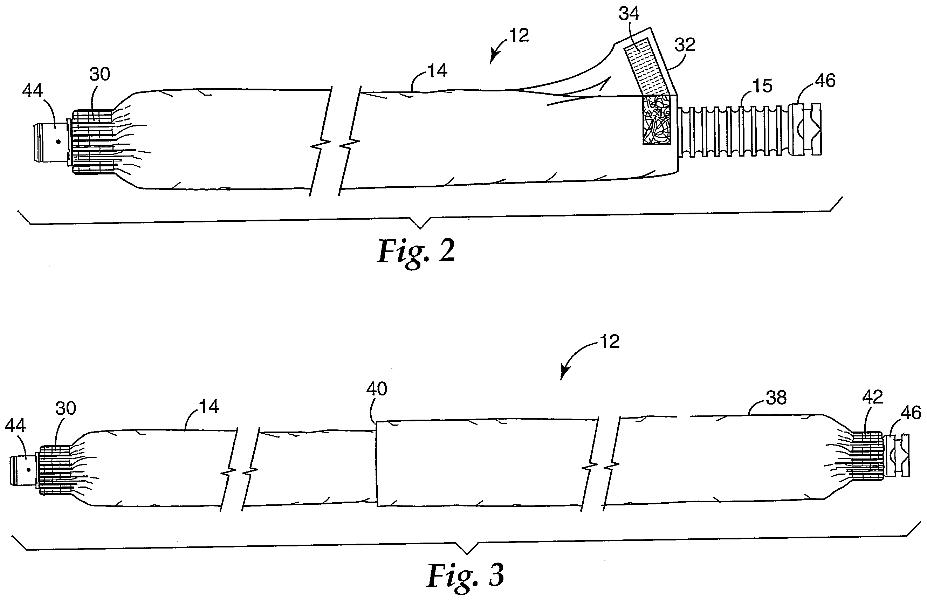

FIG. 1 shows a powered air purifying respirator system **10** that includes an adjustable length hose **12** that has a sleeve **14** disposed over a tube **15** (FIG. 2) through which air passes from a filtering unit **16** to a hood **18.** The filtering unit **16** has a fan **20** that is driven by an electric motor **22.** The fan **20** draws air through a filter element **24** and forces that filtered air into the interior gas space defined by the hood **18.** The fan **20** and the electric motor **22** are typically contained within a housing **26.** An electric switch **28** may be included in the circuit **31** to enable the PAPR **10** to be turned on and off. The filter element is commonly contained within a filter cartridge housing **24,** which housing **24** is usually removably attached to the PAPR housing **26.** The PAPR housing **26** is commonly worn about the waist of the user and may be supported by a belt like the one shown in US Patent Des. D501,288 to Taylor. Examples of a PAPR systems in which the present invention may be used are shown in U.S. Patents 6,796,304 to Odell et al., 6,666,209 to Bennett et al., and 6,575,165 to Cook et al. Filter media that could be used to provide the wearer with a source of clean air include particulate filter media such as a webs of electrically charged fibers, particularly electrically charged microfibers - see US Patents 6,783,574 to Angadjivand et al., 6,119,691 to Angadjivand et al., and 5,472,600 to Ellefson et al. Alternatively, gaseous filters such as activated carbon filters can also be used to filter gaseous contaminants - see U.S. Patents 6,344,071 B1 to Smith et al., 5,078,132 to Braun et al., and 4,443,354 to Eian. Filter elements that contain combinations of particulate and gaseous filter media may also be used in the filter cartridge(s). Multiple cartridges **24** also may be attached to the filtering unit **16.** The PAPR also could be provided with a sensor that tells the user when the flow rate has dropped below a specified level - see U.S. Patent 6,615,828 to Petherbridge. In addition, a nonvolatile memory device may be associated with the filter element to keep a record of the filter element's usage - see US Patent 6,186,130 to Hogue. A system of calibrating air flow may also be used - see U.S. Patent 6,666,209 B2 to Bennett et al. Although the PAPR shown in FIG. 1 uses a hood **18** as a face piece that provides an interior gas space separate from an exterior gas space, a supplied air system in accordance with the present invention also could use a face mask that fits over wearer's nose and mouth (and optionally the eyes) or it could be a full containment suit - see, for example, U.S. Patent 6,286,144 to Henderson et al.

FIG. 2 shows a first embodiment of an adjustable length hose **12** in accordance with the present invention. Adjustable length hose **12** includes a first sleeve **14** and a tube **15.** The sleeve **14** is disposed over the tube **15** and has first and second ends **30** and **32,** respectively. The first end **30** is elasticated, that is, it can be resiliently stretched to accommodate and snugly grasp the first end **44** of the tube **15.** The sleeve end **30** also can be enlarged so that the tube **15** can be passed therethrough when assembling the device. The elastic nature of end **30** allows the sleeve **14** to fit snugly and tightly about the tube **15.** Any other method that permits adequate securement (physical, mechanical, or chemical) of the first sleeve end **30** to the first tube end **44** is contemplated under this invention. Besides mechanical gripping, the sleeve and could be, for example, adhered (using an adhesive) welded (using similar plastics) or fastened (using buttons or snaps) to the tube end.

As FIG. 2 shows, the opposing end **32** of sleeve **14** has a fastener **34** that allows the size of the sleeve opening at end **32** to be adjusted. The fastener **34** may be of the hook and loop type. The tube **15** may be made from a plastic material that is disposed over a spirally wound cord (see, for example, UK Patent 1,419,841). This kind of tube **15** is flexible and tends to exhibit resistance to axial compression. The tube **15** is formed in such a way that displays a tendency to expand in the axial direction if compressed from its naturally extended state. Conversely, if the tube is extended axially by exerting tension on it, it has a tendency to compress to return to its naturally extended state. The spirally wound cord provides shape and structural integrity to the tube **15** and allows it to be extended or compressed as needed. Alternatively, the tube could be a flexible, molded convoluted hose. The first end **44** of tube **15** can have, for example, a quick release swivel (Q. R. S.) adapter on it so that the hose **12** can be attached to the facepiece **18.** The opposing end **46** of the hose **12** can have a bayonet fitting for being secured to the filtering unit **16** (FIG. 1). Other adapters or fittings may be used as desired. A portion of the tube **15** extends out from the interior of sleeve **14** to achieve the desired length. The fastener 34 is tightened around the tube **15** to preclude additional tube **15** from being extended (or withdrawn) from within the sleeve **14.** The length of the hose **12** is thus set. Because of its spirally-wound or helix, cord-like structure, the tube **15** has a tendency to expand axially to its full unencumbered length. By having the sleeve elastically secured to the tube at end **30** and by having the fastener **34** tightly secured about the tube at end **32,** the natural expansion of the tube **15** to its full-length can be retarded, and thus a desired length can be achieved. Although a mechanical fastener has been illustrated in the drawing, any technique for retarding hose expansion is contemplated under this invention. The sleeve **14,** for instance, can be secured to the tube **15** through use of essentially any gripping device such as an elastic fitting, an adhesive tape, a button, etc. or through the techniques mentioned above with respect to the first sleeve end.

FIG. 3 shows that the adjustable length hose **12** may include a second sleeve **38** that has first and second ends **40** and **42.** The first end **40** is open and is sized to be sufficiently large to accommodate the first sleeve **14.** The second end **42** is elasticated to fit tightly about the tube **15** (FIG. 2). The second sleeve **38** allows essentially the full-length of the tube to be uniform in appearance and fully protected or covered by an outer sleeve member.

To assemble the adjustable length hose **12** of the first embodiment of the present invention illustrated in FIGs. 2 and 3, the user starts by inserting the first end **44** of the tube **15** into the second end **32** of the first sleeve **14.** The tube **15** is drawn through the sleeve **14** until the elasticated end **30** fits tightly about the tube **15** and the first end **44** of the tube **15** projects axially from the sleeve **14.** The tube **15** is compressed within the sleeve **14** until the desired length is achieved. The fastener **34** is secured around the tube **15** at the desired length. The second end **46** of the tube **15** is placed in the open end **40** of the second sleeve **38.** The second end **42** of sleeve **38** is secured snugly about the second end **46** of the tube **15.** This can be achieved by making end **42** of tube **38** elasticated or by providing it with an adjustable fastener such as a hook and loop material. The hose **12** is thus adjusted to the desired length to fit the particular user, and the tube **15** is fully covered by the first and second sleeves **14** and **38.** The inventive adjustable length hose thus precludes excessive hose slack from occurring during use of the device by a smaller user. The annoyance and potential hazard that the slack creates is therefore reduced. To further reduce any opportunity for the hose to become inadvertently caught on an adjacent object, hardware could also be provided on the system, which hardware allows the hose to be held closely to the wearer's body when in use.

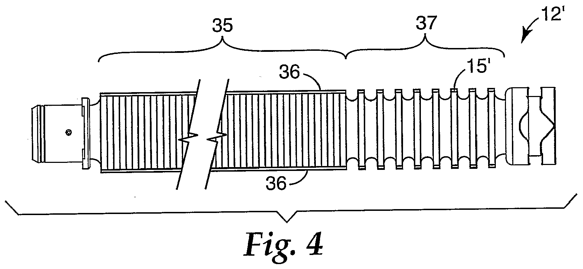

FIG. 4 shows another method of providing an adjustable length hose **12'.** In this embodiment, the tube **15'** is precluded from achieving its natural expansion through use of a bonding material **36.** Alternatively, the tube could be a molded, convoluted tube that expands axially, not naturally, but in response to tension (or pulling) by the user in the axial direction. The bonding material **36** may be in the form of a bead that extends as a line along the side of the tube **15'.** The bonding material may extend along the full length of the side of the tube or along the side a distance sufficient to allow for expansion to accommodate users of various sizes. Two or more such bead lines may be disposed on the tube **15'** at 180° to each other. If desired, four bead lines could be used at 90° to each other. To achieve a hose **12'** of a desired length, the hose **15'** can be secured at one end and pulled at the other to break the bead lines **36.** When the hose **15'** has been extended to its desired length, the tension on the hose should cease so that the bead lines **36** are not broken further. The extended hose has two portions, a first compressed portion **35** and a second extended portion **37.** The desired length of the hose **12'** is thus achieved.

In addition to the adjustment mechanisms illustrated and described above, other approaches are contemplated in connection with the present invention. Hose length could, for example, be adjusted using one or more the following techniques: (i) molded concertina or corrugations, like in a flexible drinking straw (see, US Patents 4,923,083, 4,078,692, and 3,346,187), which corrugations can be extended to a fixed length by pulling on the hose in the axial direction (ii) internal frictionally engaging mechanism that holds the internal structure of the hose in a compressed state or desired length via, for example, a ribbed outer edge that engages the internal structure of the tube material; (iii) internal adjustable length wire that extends through the central portion of the hose to connect opposing ends of the hose together (by varying the length of the wire thus controls the length of the breathing hose); (iv) telescopic device, (like an antenna) that can be adjusted by sliding the structured members to the correct length to suit the user; and (v) recoil hose stored under tension in a housing (the hose can be withdrawn from the housing to the desired length). Additionally, a brace and clip arrangement, which uses a flexible bracing arm, could be fixed on a portion of the compressed flexible hose. The clips would hold the brace in place to engage the hose at a particular corrugation(s) and keep the hose from extending between the braced points.

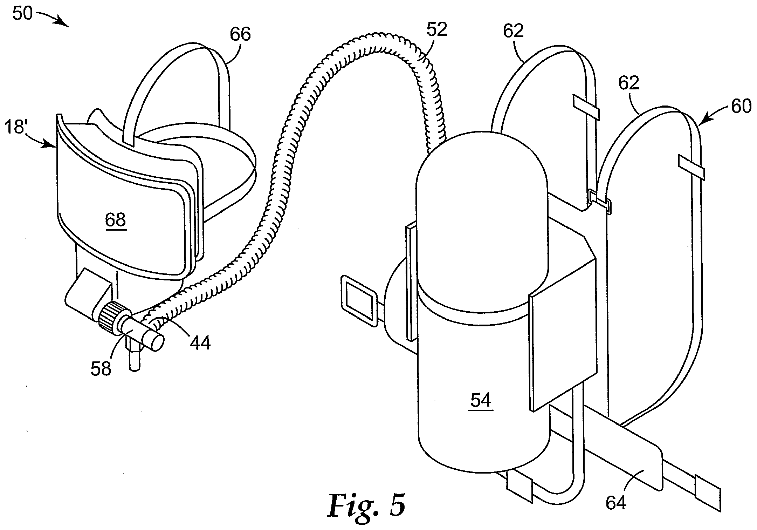

FIG. 5 illustrates a SCBA system **50** that includes a facepiece **18',** a hose **52,** and a clean air supply source or tank **54.** The hose **52** is attached at its first end **44** to a fitting **58** that is attached to facepiece **18'.** The second end of the hose **52** is secured to a fitting that is connected to the tank **54.** The tank **54** contains a pressurized supply of clean air for a wearer of the SCBA to breathe. The SCBA can be supported on a wearer's back through use of a harness **60** that includes straps and **62** and a belt **64.** The facepiece **18'** is adapted to fit over the nose, mouth, and eyes of the wearer and includes a harness **66** for supporting the facepiece **18'** on the wearer's head. The facepiece **18'** also has a window **68** through which the wearer can see the surrounding environment. The hose **52** can be adjusted in length using any one of the methods or systems described above.

Although the above discussion describes clean breathable air being supplied by either a PAPR or SCBA, it may also be possible for the clean air to be supplied to the user's facepiece or headtop through an air regulator that supplies clear air to the user from either low or high pressure systems. In such a device, the user typically wears the regulator on a belt, the connection being made between the regulator and the facepiece / headtop by the hose described above. The regulator receives its air supply through a flexible adjustable length hose from either a lower or high pressure supply where low pressure typically comes from an air pump and where high pressure typically comes from an air compressor. A known regulator product has been marketed under the brand Flowstream™ by 3M, and such products have used wide bore hose (diameter ≥ about 3 cm, typically about 2 to 5 cm) for the connection between the regulator and the facepiece / headtop. For purposes of this invention, a hose that is connected to a clean air supply source through a regulator or other device is considered to be a hose that "extends from" the clean air supply source to the facepiece.

Hoses that have been used in the past had a fixed length that would accommodate persons of essentially all sizes. This length is referred to in this document as the "conventional hose length" or "CHL". The present invention is able to provide a variable or desired hose length "DHL", which is equivalent to the CHL minus "X" where "X" is the amount of unneeded hose length, which would have created the extra slack in previously known systems. Conventional hose length typically was about 100 centimeters (cm). This hose length was provided to accommodate users of about 150 to 190 cm tall. For the smallest users, the minimum DHL is about 60cm and therefore X=40cm. The DHL for each user of the invention thus resides somewhere between the CHL of about 100cm and about 60cm. Thus, the hose of the invention is preferably adjustable from about 60 cm in length to 100 cm in length.

The above discussion has for the most part described the hose adjustment apparatus as being a feature associated with or fitted to the hose itself, but this device may, however, also be associated or fitted to the facepiece. This could be accomplished by fashioning the facepiece with a device that allows for entry of the hose into the facepiece. Such a device could be particularly suitable for facepieces that are in the form of hoods or headtops (helmets). Likewise, the adjustment feature could also be associated with or fitted to the clean air supply source.

This invention may take on various modifications and alterations without departing from its scope. This invention, accordingly, is not to be limited to the above-described but is to be controlled by the limitations set forth in the following claims.

This invention also may be suitably practiced in the absence of any element not specifically disclosed herein.

## Claims

1. A supplied air respirator (10) that comprises:
(a) a clean air supply source (54) that is designed to be carried by a user of the supplied air respirator (10);
(b) a face piece (18; 18') that is sized to fit at least over the user's nose and mouth; and
(c) an adjustable length hose (12; 12'; 52) that extends from the clean air supply source (54) to the facepiece (18; 18') for supplying clean air to the user, **characterised in that** the adjustable length hose (12; 12'; 52) includes a first sleeve (14) and a tube (15; 15'), the first sleeve (14) being disposed over the tube (15; 15') and having first and second ends (30, 32), wherein the first end (30) engages the first end (44) of the tube (15; 15'), and wherein the second sleeve end (32) firmly grasps the tube (15; 15') in a spaced relation to the first sleeve end (30), wherein a portion of the tube (15) extends out from the interior of sleeve (14) to achieve the desired length of the adjustable length hose (12; 12'; 52).

2. The supplied air respirator (10) of claim 1, wherein the adjustable length hose (12; 12'; 52) comprises a tube (15; 15') that has a bonding material (36) secured to a side of the tube (15; 15'), the bonding material (36) precluding the tube (15; 15') from achieving a natural expansion, and wherein the bonding material (36) is broken to allow the tube (15; 15') to expand to achieve a desired tube length.

3. The supplied air respirator (10) of claim 1, wherein the hose length is adjustable through use of molded corrugations that may be extended to a desired length by pulling in the axial direction of the hose (12; 12'; 52).

4. The supplied air respirator (10) of claim 1, wherein the hose (12; 12'; 52) comprises an internal frictionally engaging mechanism that holds the hose (12; 12'; 52) to a desired length.

5. The supplied air respirator (10) of claim 1, wherein the hose (12; 12'; 52) comprises an internal adjustable length wire that extends centrally through an expandable length tube (15; 15') to set the tube (15; 15') to a desired length.

6. The supplied air respirator (10) of claim 1, wherein the adjustable length hose (12; 12'; 52) includes a recoil means that allows the hose (12; 12'; 52) to be withdrawn from a housing (26) to a desired length.

7. The supplied air respirator (10) of claim 1, wherein the adjustable length hose (12; 12'; 52) includes a tube (15; 15') that has a tendency to expand in its axial direction when compressed and further comprises a bracing clip arrangement that engages the tube (15; 15') at a particular location to keep it from extending between braced points.

## Patentansprüche

1. Umluftabhängiges Atemschutzgerät (10), das Folgendes umfasst:
(a) eine Reinluft-Versorgungsquelle (54), die dazu ausgelegt ist, von einem Benutzer des umluftabhängigen Atemschutzgeräts (10) getragen zu werden;
(b) ein Gesichtsteil (18; 18'), das bemessen ist, um mindestens über Nase und Mund des Anwenders zu passen; und
(c) einen Schlauch mit einstellbarer Länge (12; 12'; 52), der sich von der Reinluft-Versorgungsquelle (54) zu dem Gesichtsteil (18; 18') erstreckt, um dem Benutzer Reinluft zuzuführen, **dadurch gekennzeichnet, dass** der Schlauch mit einstellbarer Länge (12; 12'; 52) eine erste Hülle (14) und ein Rohr (15; 15') einschließt, wobei die erste Hülle (14) über dem Rohr (15; 15') angeordnet ist und erste und zweite Enden (30, 32) aufweist, wobei das erste Ende (30) in das erste Ende (44) des Rohrs (15; 15') eingreift, und wobei das zweite Hüllenende (32) das Rohr (15; 15') beabstandet zum ersten Hüllenende (30) fest umgreift, wobei sich ein Abschnitt des Rohrs (15) aus dem Inneren der Hülle (14) heraus erstreckt, um die gewünschte Länge des Schlauchs mit einstellbarer Länge (12; 12'; 52) zu erzielen.

2. Umluftabhängiges Atemschutzgerät (10) nach Anspruch 1, wobei der Schlauch mit einstellbarer Länge (12; 12'; 52) ein Rohr (15; 15') umfasst, das ein an einer Seite des Rohrs (15; 15') befestigtes Verbindungsmaterial (36) aufweist, wobei das Verbindungsmaterial (36) das Rohr (15; 15') am Erreichen einer natürlichen Ausdehnung hindert, und wobei das Verbindungsmaterial (36) zerbrochen wird, um zu ermöglichen, dass sich das Rohr (15; 15') ausdehnt, um eine gewünschte Rohrlänge zu erreichen.

3. Umluftabhängiges Atemschutzgerät (10) nach Anspruch 1, wobei die Schlauchlänge durch die Verwendung von eingeformten Wellungen verstellbar ist, die durch Ziehen in axialer Richtung des Schlauchs (12; 12'; 52) auf eine gewünschte Länge ausgedehnt werden können.

4. Umluftabhängiges Atemschutzgerät (10) nach Anspruch 1, wobei der Schlauch (12; 12'; 52) einen internen Reibeingriffsmechanismus umfasst, der den Schlauch (12; 12'; 52) auf einer gewünschten Länge hält.

5. Umluftabhängiges Atemschutzgerät (10) nach Anspruch 1, wobei der Schlauch (12; 12'; 52) einen internen Draht mit verstellbarer Länge umfasst, der sich zentral durch ein Rohr (15; 15') mit ausdehnbarer Länge erstreckt, um das Rohr (15; 15') auf eine gewünschte Länge einzustellen.

6. Umluftabhängiges Atemschutzgerät (10) nach Anspruch 1, wobei der Schlauch mit einstellbarer Länge (12; 12'; 52) ein Rückspringmittel einschließt, das es ermöglicht, dass der Schlauch (12; 12'; 52) aus einem Gehäuse (26) auf eine gewünschte Länge zurückgezogen wird.

7. Umluftabhängiges Atemschutzgerät (10) nach Anspruch 1, wobei der Schlauch mit einstellbarer Länge (12; 12'; 52) ein Rohr (15; 15') einschließt, das eine Tendenz aufweist, sich bei Kompression in seiner axialen Richtung auszudehnen, und weiter eine Versteifungsclipanordnung umfasst, die an einer bestimmten Stelle in das Rohr (15; 15') eingreift, um es an einer Ausdehnung zwischen versteiften Punkten zu hindern.

## Revendications

1. Respirateur à air fourni (10) qui comprend :
(a) une source d'alimentation en air propre (54) qui est conçue pour être portée par un utilisateur du respirateur à air fourni (10) ;
(b) un élément facial (18 ; 18') qui est dimensionné pour s'ajuster au moins par-dessus le nez et la bouche de l'utilisateur ; et
(c) un tuyau flexible de longueur réglable (12 ; 12' ; 52) qui s'étend de la source d'alimentation en air propre (54) jusqu'à l'élément facial (18 ; 18') pour amener de l'air propre à l'utilisateur, **caractérisé en ce que** le tuyau flexible de longueur réglable (12 ; 12' ; 52) inclut un premier manchon (14) et un tube (15 ; 15'), le premier manchon (14) étant disposé par-dessus le tube (15 ; 15') et ayant des première et deuxième extrémités (30, 32), dans lequel la première extrémité (30) vient en prise avec la première extrémité (44) du tube (15 ; 15'), et dans lequel la deuxième extrémité de manchon (32) saisit fermement le tube (15 ; 15') dans une relation espacée par rapport à la première extrémité de manchon (30), dans lequel une partie du tube (15) s'étend hors de l'intérieur du manchon (14) pour réaliser la longueur souhaitée du tuyau flexible de longueur réglable (12 ; 12' ; 52).

2. Respirateur à air fourni (10) selon la revendication 1, dans lequel le tuyau flexible de longueur réglable (12 ; 12' ; 52) comprend un tube (15 ; 15') qui a un matériau de liaison (36) fixé à un côté du tube (15 ; 15'), le matériau de liaison (36) empêchant le tube (15 ; 15') de réaliser une expansion naturelle, et dans lequel le matériau de liaison (36) est rompu pour permettre au tube (15 ; 15') de s'expanser pour réaliser une longueur de tube souhaitée.

3. Respirateur à air fourni (10) selon la revendication 1, dans lequel la longueur du tuyau flexible est réglable par l'utilisation de striures moulées qui peuvent être étendues à une longueur souhaitée en tirant dans la direction axiale du tuyau flexible (12 ; 12' ; 52).

4. Respirateur à air fourni (10) selon la revendication 1, dans lequel le tuyau flexible (12 ; 12' ; 52) comprend un mécanisme interne de mise en prise par frottement qui maintient le tuyau flexible (12 ; 12' ; 52) à une longueur souhaitée.

5. Respirateur à air fourni (10) selon la revendication 1, dans lequel le tuyau flexible (12 ; 12' ; 52) comprend un fil interne de longueur réglable qui s'étend au centre à travers un tube de longueur expansible (15 ; 15') pour mettre le tube (15 ; 15') à une longueur souhaitée.

6. Respirateur à air fourni (10) selon la revendication 1, dans lequel le tuyau flexible de longueur réglable (12 ; 12' ; 52) inclut un moyen de détente qui permet au tuyau flexible (12 ; 12' ; 52) d'être retiré d'un logement (26) jusqu'à une longueur souhaitée.

7. Respirateur à air fourni (10) selon la revendication 1, dans lequel le tuyau flexible de longueur réglable (12 ; 12' ; 52) inclut un tube (15 ; 15') qui a tendance à s'expanser dans sa direction axiale lorsqu'il est comprimé et comprend en outre un agencement d'attache de serrage qui vient en prise avec le tube (15 ; 15') à un emplacement particulier pour l'empêcher de s'étendre entre les points serrés.
